# EUROPÄISCHE PATENTSCHRIFT

(11) **EP 2 145 555 B1**
(45) Veröffentlichungstag und Bekanntmachung des Hinweises auf die Patenterteilung: **25.08.2010**
(21) Anmeldenummer: 08012900.0
(22) Anmeldetag: 17.07.2008
(51) Int. Cl.: A44B 18/00, A61F 13/62

(54) **Verbundstoffelement für einen Klettverschluss, insbesondere für einen Windelverschluss**
Compound material element for a hook-and-loop fastener, in particular for a nappy closure
Elément composite pour une fermeture à crochet, notamment pour fermeture de couche

(43) Veröffentlichungstag der Anmeldung: 20.01.2010
(73) Patentinhaber: Nordenia Technologies GmbH, 48599 Gronau (DE)
(72) Erfinder: Baldauf, Georg, 48366 Laer (DE); Homölle, Dieter, 48607 Ochtrup (DE)
(74) Vertreter: Albrecht, Rainer Harald

(56) Entgegenhaltungen:
- EP-A- 1 690 464
- EP-A- 1 690 967
- DE-A- 19 722 748
- DE-A-102004 053 469

## Beschreibung

Die Erfindung betrifft ein Verbundstoffelement für einen Klettverschluss, insbesondere für einen Windelverschluss, bestehend aus einem textilen Träger und einer auf den Träger aufkaschierten textilen Funktionsschicht für den Eingriff von Kletthaken.

Klettverschlüsse sind hinlänglich bekannt. Sie bestehen im Wesentlichen aus einer (männlichen) Verschlusskomponente mit Kletthaken und einer (weiblichen) Komponente, in die die Kletthaken eingreifen. Verschlüsse dieser Art sind kostengünstig herzustellen, sind leicht zu bedienen und lassen sich wiederholt verschleißfrei öffnen und schließen. Daher werden unter anderem Windeln mit solchen Klettverschlüssen ausgerüstet. Im Gegensatz zu alternativ eingesetzten Klebeverschlüssen bieten Klettverschlüsse an Windeln den Vorteil, dass sie auch bei Kontakt mit beispielsweise einem Babyöl oder einer Hautcreme weiterhin verschließbar sind.

Ein Verbundstoffelement, welches den weiblichen Verschlussteil insbesondere eines Windelklettverschlusses bildet, wird beispielsweise in EP 1 579 779 B1 offenbart. Dabei handelt es sich um ein Verbundstoffelement, bei dem auf einer Trägerfolie ein textiles Material aufgebracht wird, welches oberflächlich Schlaufen zur Verankerung der Kletthaken aufweist. Üblicherweise werden derartige Verbundstoffetemente im Bereich des Windelbündchens angeordnet, so dass ein Verschließen der Windel mit geeigneten Verschlussbändem erfolgen kann, weiche die erforderlichen Kletthaken aufweisen. Die dort beschriebenen Verbundstoffelemente verfügen über eine angenehm weiche und somit hautfreundliche Haptik. Aufgrund der Trägerfolie ist die Verschlusskomponente des Klettverschlusses jedoch nicht atmungsaktiv.

Noch ein Verbundstoffelement für einen Kletterverschluss wird in EP-1690967 offenbart. Es handelt sich um ein Verbundstoffelement für einen Windelklettverschluss, das aus einen textilen Träger und einer auf den Träger aufkaschierten textilen Funktionsschicht für den Eingriff von Kletthaken besteht.

Der Erfindung liegt daher die Aufgabe zugrunde, ein Verbundstoffelement für einen Windelverschluss anzugeben, dessen Atmungsaktivität verbessert ist, ohne dass dabei optische, haptische und funktionelle Eigenschaften beeinträchtigt sind. Diese Aufgabe wird durch ein Verbundstoffelement gemäß Anspruch 1 gelöst, bei dem der textile Träger und die textile Funktionsschicht miteinander verklebt sind und einen luftdurchlässigen Verbund bilden. Dabei ist der Klebstoff in einem Muster auf dem Träger appliziert, das einen Klebstoffrahmen mit vollflächigem Klebstoffauftrag und innerhalb des Klebstoffrahmens eine Klebestruktur aus regelmäßig angeordneten Klebeflächen und klebstofffreien Bereichen aufweist. Außerdem bildet der Klebstoffrahmen den Rand des Verbundstoffelementes. Das Verkleben bzw. die Kaschierung kann dabei mit einem reaktiven PUR-Klebstoff oder einem Schmelzkleber erfolgen.

Die Kombination aus zwei ausschließlich textilen und daher jeweils luftdurchlässigen Schichten, die miteinander so verklebt sind, dass der Verbund klebstofffreie Bereiche aufweist, ist insgesamt ebenfalls luftdurchlässig. Dadurch wird der Kaschierverbund atmungsaktiv und eignet sich besonders für den Einsatz als weibliches Verschlusselement einer Windel. Die Funktion zur Verankerung von Kletthaken eines (männlichen) Verschlussbandes wird durch das teilflächige Verkleben der Funktionsschicht mit dem textilen Träger insofern begünstigt, als die Kletthaken tief in die Funktionsschicht eingreifen können. Das vollflächige Verkleben des Randbereichs des erfindungsgemäßen Verbundstoffelementes verhindert ein randseitiges Aus- oder Abreißen des Textils beim Öffnen des Verschlusses. Auf diese Weise wird eine gute Haltbarkeit des Verschlusses erreicht, so dass der Verschluss häufig benutzt werden kann. Außerdem werden sich die Kletthaken des Verschlussbandes in den verklebten Randbereichen des Verbundstoffelementes weniger intensiv verhaken, wodurch das Material beim Öffnen des Verschlusses einer geringeren Beanspruchung ausgesetzt ist. Auch das trägt dazu bei, dass das erfindungsgemäße Verbundstoffelement auch bei häufigem Gebrauch nicht ausfranst und seine Funktionsfähigkeit auch in den Randbereichen behält.

In möglichen Ausgestaltungen des erfindungsgemäßen Verbundstoffelementes kann vorgesehen sein, dass der textile Träger aus einem Spinnvlies besteht, der bevorzugt ein Flächengewicht von 10 bis 50 g/m² aufweist. Dadurch wird erreicht, dass das Verbundstoffelement ein geringes Gewicht hat. Diese Eigenschaft ist bei Windelprodukten insofern wichtig, als diese zumeinst in großen Stückzahlen angeschafft und entsorgt werden müssen. Ein möglichst geringes Gewicht stellt dabei einen entscheidenden Vorteil dar. Das Spinnvlies kann opak, weiß oder farbig sein.

Es kann außerdem vorgesehen sein, dass der textile Träger aus Fasern aus Polyolefinen (beispielsweise PP; PE), Polyolefin-Copolymeren, Polyamiden (PA) oder Polyestern (beispielsweise PET) besteht. Die haptischen Eigenschaften von derartigen Textilien hinterlassen beim Benutzer des Verschlusses einen weichen und hautfreundlichen Eindruck. In Verbindung mit der erfindungsgemäßen Luftdurchlässigkeit des Verbundstoffes werden die Windelverschlusskomponenten bei einem Hautkontakt daher kaum als störend empfunden.

Gemäß einer bevorzugten Variante des erfindungsgemäßen Verbundstoffelementes besteht die textile Funktionsschicht aus einer Kettwirkware, bei der durch ein wirktechnisches Verfahren textile Kettenstränge und Schlaufen gebildet sind, die sich in besonderer Weise für den Eingriff der Kletthaken eignen. Beim Schließen des Verschlusses ist es daher möglich, dass eine möglichst große Anzahl an Kletthaken in eine Schlaufe oder ähnliches eingreift, wodurch der Verschluss weniger leicht zu öffnen ist. Alternativ zu der Kettwirkware kann die Funktionsschicht auch aus einem Gewebe oder einem Vliesstoff bestehen, wodurch sich der gesamte Kaschierverbund flauschig anfühlt und auf der Haut weniger kratzt. Der Vliesstoff kann beispielsweise aus PA, PET oder PP gebildet sein. Eine Windel mit flauschigen Bestandteilen wird gemeinhin als komfortabel und tragefreundlich empfunden.

Die Klebeflächen bilden innerhalb des Klebstoffrahmens insbesondere eine streifen-, gitter-, punkt- oder zellenförmige Struktur, wobei vorgesehen sein kann, dass der Anteil der Klebeflächen innerhalb des Klebstoffrahmens 10 bis 70 %, vorzugsweise 40 bis 60 % bezogen auf die vom Klebstoffrahmen eingeschlossene Fläche beträgt. Die Bildung von unverklebten textilen Bereichen fördert die Luftdurchlässigkeit des Kaschierverbundes und wirkt sich gleichzeitig günstig auf die Verschlusswirkung aus. Letzteres hängt damit zusammen, dass die Kletthaken eines Verschlussbandes in einem nicht verklebten textilen Bereich der Funktionsschicht nicht nur an der Ober seite der Schicht Schlaufen oder Ähnliches antreffen, sondern auch innerhalb der Schicht und auf ihrer nicht verklebten Unterseite. Sowohl der Anteil der verklebten Fläche an der Gesamtfläche des Stoffverbundes als auch die Form oder die Art der Verklebung hängt dabei von der Wahl des Materials und den wirkenden Öffnungskräften ab. Ist der Anteil der verklebten Fläche größer als 70 %, kann beispielsweise als Funktions- oder Trägertextil ein Spinnvlies-Meltblown-Spinnvlies-Verbund (SMS) aus Polypropylen mit einem Flächengewicht von 10 bis 20 g/m² vorgesehen sein.

Im Folgenden wird die Erfindung anhand einer lediglich ein Ausführungsbeispiel dar stellenden Zeichnung näher erläutert. Die Figuren zeigen:
- **Fig. 1**: einen vertikalen Schnitt durch ein Verbundstoffelement,
- **Fig. 2**: eine Aufsicht auf ein Verbundstoffelement mit einer parallel zur Schmalseite angeordneten, streifenförmigen Kleberstruktur und
- **Fig. 3**: eine Aufsicht auf ein Verbundstoffelement mit einer diagonal angeordneten, streifenförmigen Kleberstruktur.

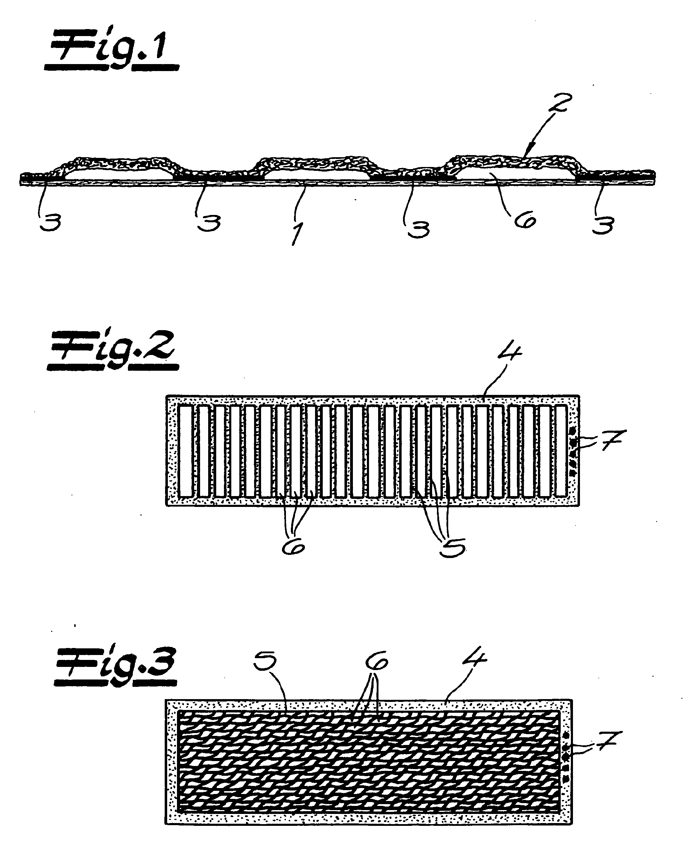

Der Fig. 1 ist ein vertikaler Schnitt durch ein Verbundstoffelement für einen Klettverschluss, insbesondere für einen Windelverschluss, zu entnehmen. Es besteht aus einem textilen Träger 1 und einer auf dem Träger 1 aufkaschierten textilen Funktionsschicht 2 für den Eingriff von Kletthaken. Dabei sind der textile Träger 1 und die textile Funktionsschicht 2 miteinander verklebt und bilden einen luftdurchlässigen Verbund, wobei der Klebstoff 3 in einem Muster auf dem Träger 1 appliziert ist, das einen Klebstoffrahmen 4 mit vollflächigem Klebstoffauftrag und innerhalb des Klebstoffrahmens eine Klebestruktur aus regelmäßig angeordneten Klebeflächen 5 und klebstofffreien Bereichen 6 aufweist. Der Klebstoffrahmen 4 bildet den Rand des Verbundstoffelementes. Der textile Träger 1 kann beispielsweise aus einem Spinnvlies bestehen, das vorzugsweise ein Flächengewicht von 10 bis 50 g/m² aufweist.
Es kann auch vorgesehen sein, dass der textile Träger 1 aus Fasern aus Polyolefinen, Polyolefin-Copolymeren, Polyamiden oder Polyestern besteht. Die textile Funktionsschicht 2 kann beispielsweise aus einer Kettwirkware, einem Gewebe oder einem Vliesstoff bestehen. Der Figur ist zu entnehmen, dass die Funktionsschicht 2 an den nicht verklebten Bereichen 6 sowohl oberseitig als auch unterseitig mit Kletthaken eines Verschlussbandes wechselwirken kann. Durch die gegebene Flexibilität der Funktionsschicht 2 an diesen Stellen wird dieser Effekt verstärkt, da beispielsweise auch Gamkomponenten aus der Mitte der Funktionsschicht von einem Kletthaken durchgriffen werden können. Ein übermäßiges Ausreißen oder Ausfransen der Funktionsschicht 2 wird dadurch verhindert, dass die Funktionsschicht 2 über die gesamte Fläche des Kaschierverbundes in regelmäßigen Abständen mit dem textilen Träger 1 fest verklebt ist.

In der Fig. 2 ist eine Aufsicht auf ein Ausführungsbeispiel des erfindungsgemäßen Verbundstoffelementes zu sehen. Das Klebestreifen-Muster ist so gewählt, dass die Klebeflächen 5 eine streifenförmige Struktur bilden. Die klebstofffreien Flächen 6 bilden ebenfalls Streifen, wobei die Streifen gemäß der Fig. 2 parallel zu der Schmalseite des Verbundstoffelementes angeordnet sind. Die Fläche ist umfangseitig mit einem Klebstoffrahmen 4 versehen. Dieser randseitige Rahmen 4 verhindert ein Ausreißen oder Ausfransen der textilen Materialien beim Öffnen des Klettverschlusses. Der Anteil aller Klebeflächen 5 innerhalb des Klebstoffrahmens beträgt 10 bis 70 % bezogen auf die von dem Klebstoffrahmen 4 eingeschlossene Fläche. Der Anteil der verklebten Flächen richtet sich nach den Kräften, die beim Öffnen des Verschlusses durch das Klettband auf den Kaschierverbund übertragen werden. Häufig ist ein Anteil der Klebeflächen bezogen auf die vom Klebstoffrahmen eingeschlossene Fläche von 40 bis 60 % vorteilhaft.

Ein Verbundstoffelement mit kleineren klebstofffreien Flächen 6, also mit einem größeren Anteil der verklebten Fläche, ist in Fig. 3 dargestellt. Das Streifenmuster ist hier derart angeordnet, dass sich eine Gitterstruktur ergibt. Alternativ zu den Streifen oder zu dem Gitter können auch andere Muster vorgesehen sein, so dass sich beispielsweise eine punkt-, zellen- oder wellenförmige Struktur ergibt. Im Hinblick auf die Fertigung und Weiterverarbeitung des Verbundstoffelementes kann vorgesehen sein, dass es mit einer Markierung 7 bedruckt wird, die eine Ablängung oder Applikation des Stoffelementes steuert. Eine solche Rapportmarkierung 7 kann wahlweise mit einem sichtbaren Farbstoff aufgedruckt sein oder alternativ mit einem Farbstoff, der erst bei einer Beleuchtung mit UV-Licht sichtbar wird.

## Patentansprüche

1. Verbundstoffelement für einen Klettverschluss, insbesondere für einen Windelverschluss, bestehend aus einem textilen Träger (1) und einer auf den Träger aufkaschierten textilen Funktionsschicht (2) für den Eingriff von Kletthaken **dadurch gekennzeichnet, dass**
der textile Träger (1) und die, textile Funktionsschicht (2) miteinander
verklebt sind und einen luftdurchlässigen Verbund bilden,
der Klebstoff (3) in einem Muster auf dem Träger (1) appliziert ist, das
einen Klebstoffrahmen (4) mit vollflächigem Klebstoffauftrag und innerhalb des Klebstoffrahmens (4) eine Klebestruktur aus regelmäßig angeordneten Klebeflächen (5) und klebstofffreien Bereichen (6) aufweist, und
der Klebstoffrahmen (4) den Rand des Verbundstoffelementes bildet.

2. Verbundstoffelement nach Anspruch 1, **dadurch gekennzeichnet, dass** der textile Träger (1) aus einem Spinnvlies besteht.

3. Verbundstoffelement nach Anspruch 2, **dadurch gekennzeichnet, dass** das Spinnvlies ein Flächengewicht von 10 bis 50 g/m² aufweist.

4. Verbundstoffelement nach einem der Ansprüche 1 bis 3, **dadurch gekennzeichnet, dass** der textile Träger (1) aus Fasern aus Polyolefinen, Polyolefin-Copolymeren, Polyamiden oder Polyestern besteht.

5. Verbundstoffelement nach einem der Ansprüche 1 bis 4, **dadurch gekennzeichnet, dass** die textile Funktionsschicht (2) aus einer Kettwirkware, einem Gewebe oder einem Vliesstoff besteht.

6. Verbundstoffelement nach einem der Ansprüche 1 bis 5, **dadurch gekennzeichnet, dass** die Klebeflächen (5) innerhalb des Klebstoffrahmens (4) eine streifenförmige, gitterförmige, punktförmige oder zellenförmige Struktur bilden.

7. Verbundstoffelement nach einem der Ansprüche 1 bis 6, **dadurch gekennzeichnet, dass** der Anteil der Klebeflächen (5) innerhalb des Klebstoffrahmens (4) 10 bis 70 %, vorzugsweise 40 bis 60 %, bezogen auf die von dem Klebstoffrahmen (4) eingeschlossenen Fläche, beträgt.

## Claims

1. A composite material element for a hook-and-loop closure, in particular for a diaper closure, consisting of a textile carrier (1) and a textile functional layer (2) laminated onto the carrier for engagement of hook-and-loop hooks, **characterised in that**
the textile carrier (1) and the textile functional layer (2) are glued to one another and form an air-permeable bond,
the adhesive (3) is applied to the carrier (1) in a pattern which comprises an adhesive frame (4) with adhesive application over the entire surface and inside the adhesive frame (4) an adhesive structure of regularly arranged adhesive areas (5) and adhesive-free regions (6), and
the adhesive frame (4) forms the edge of the composite material element.

2. The composite material element according to claim 1, **characterised in that** the textile carrier (1) consists of a spunbonded fabric.

3. The composite material element according to claim 2, **characterised in that** the spunbonded fabric has a basis weight of 10 to 50 g/m².

4. The composite material element according to any one of claims 1 to 3, **characterised in that** the textile carrier (1) consists of fibres of polyolefins, polyolefin copolymers, polyamides or polyesters.

5. The composite material element according to any one of claims 1 to 4, **characterised in that** the textile functional layer (2) consists of a tricot fabric, a woven fabric or a non-woven fabric.

6. The composite material element according to any one of claims 1 to 5, **characterised in that** the adhesive areas (5) inside the adhesive frame (4) form a strip-shaped, grid-shaped, punctiform or cells-shaped structure.

7. The composite material element according to any one of claims 1 to 6, **characterised in that** the fraction of the adhesive areas (5) inside the adhesive frame (4) is 10 to 70%, preferably 40 to 60%, relative to the area enclosed by the adhesive frame (4).

## Revendications

1. Elément en matériau composite pour bande agrippante, notamment pour fermeture de couches, composé d'un support textile (1) et d'une couche fonctionnelle textile (2) plaquée sur le support pour l'engrènement de crochets agrippants, **caractérisé en ce que**
le support textile (1) et la couche fonctionnelle textile (2) sont collés ensemble et forment un composite imperméable à l'air,
la colle (3) est appliquée sur le support (1) suivant un modèle qui présente un cadre de colle (4) avec une application de colle sur toute sa surface et à l'intérieur du cadre de colle (4) une structure collante faite de surfaces collantes (5) disposées régulièrement et de zones exemptes de colle (6) et que le cadre de colle (4) constitue le bord de l'élément en matériau composite.

2. Elément en matériau composite selon la revendication 1, **caractérisé en ce que** le support textile (1) consiste en un filé-lié.

3. Elément en matériau composite selon la revendication 2, **caractérisé en ce que** le filé-lié présente un grammage de 10 à 50 g/m².

4. Elément en matériau composite selon une des revendications 1 à 3, **caractérisé en ce que** le support textile (1) consiste en fibres de polyoléfines, copolymères de polyoléfine, polyamides ou polyesters.

5. Elément en matériau composite selon une des revendications 1 à 4, **caractérisé en ce que** la couche fonctionnelle textile (2) consiste en un tricot ajouré, un tissé ou un non-tissé.

6. Elément en matériau composite selon une des revendications 1 à 5, **caractérisé en ce que** les surfaces collantes (5) forment à l'intérieur du cadre de colle (4) une structure en forme de ruban, en forme de grille, en forme de point ou en forme de cellule.

7. Elément en matériau composite selon une des revendications 1 à 6, **caractérisé en ce que** la proportion des surfaces collantes (5) à l'intérieur du cadre de colle (4) représente 10 à 70 %, de préférence 40 à 60 %, de la surface circonscrite par le cadre de colle (4).
